# EUROPEAN PATENT APPLICATION

(11) **EP 1 360 961 A1**
(43) Date of publication of application: **12.11.2003**
(21) Application number: 02076804.0
(22) Date of filing: 07.05.2002
(51) Int. Cl.: A61K 38/08, A61K 38/10, A61K 38/16, A61K 31/41, A61K 31/00

(54) **Use of antimicrobial peptides for potentiating the activity of antimicrobial agents**

(71) Applicant: AM-Pharma B.V., 3723 MB Bilthoven (NL)
(72) Inventor: Nibbering, Petrus, Hendricus, 2215 SL Voorhout (NL); Lupetti, Antonella, 56100 Pisa (IT)
(74) Representative: Wittop Koning, Tom Hugo

(57) **Abstract**

Described is the use of a combination of at least two antimicrobial agents for the preparation of a medicament for the treatment of an infection of microbes in a subject in need thereof, the microbes being resistant to the first antimicrobial agent, the second antimicrobial agent comprising an antimicrobial peptide and a second antimicrobial agent, a medicament comprising an antimicrobial peptide for treating a microbial infection, and the use of a microbistatic agent and an antimicrobial peptide for the preparation of a microbicidic agent.

## Description

The invention relates to the use of a combination of at least two antimicrobial agents for the preparation of a medicament for the treatment of an infection of microbes in a subject in need thereof, one of the microbial agents comprising an antimicrobial peptide, to a medicament for treating an infection of microbes comprising an antimicrobial peptide and to the use of an antimicrobial peptide and a fungistatic agent for the preparation of a fungicidic agent.

These causing bacteria, yeasts, fungia and viruses are becoming increasing resistant to the effects of antibiotic medication. For certain types of resistant bacteria for example, powerful antibiotics, such as vancomycin are the treatment of last resort, that are used when other antibiotic therapies fail. Increasingly however, microbes (i.e. bacteria, fungi, yeasts and viruses) that normally respond to powerful and effective antibiotics have become resistant to their effects. Antifungal and antiviral agents are even more limited in their use as compared with antibacterial agents, as the number of antifungal, anti-yeast and antivirus agents are limited, and also fungi, yeasts and viruses become more and more resistant against the treatment with the respective antibiotic agents. For example *Candida ablicans* is an opportunistic yeast that causes mucosal and invasive infections in immunocomprised hosts. (**Revankar, S. G. et al**., J. Infect. Dis. **174:**821-827 (1996). **Revankar, S. G. et al**., Am. J. Med. **105:**7-11 (1998).)

In particular fluconazole is most frequently employed for treatment of *Candida* infections. However, long-term fluconazole treatment often induce a selective pressure which results in the developement of fluconazole-resistant *C.albicans* strains (**Rex, J.H. et al**., Antimicrob. Agents Chemother.**39:**1-8 (1995).)

The frequent isolation of microbes that are resistant to their antibiotic treatment, such as *C.albicans* strains, being resistant against fluconazole, points to a pressing need for the developement of new effective antimicrobial agents.

In the light of the need of new antimicrobial, antifungial, antiyeast and antiviral regiments, it has now been found that antimicrobial peptides have unrevealed potentials by resensibilising multidrug-resistant microbes for their antimicrobial agent that they previously have become resistant for. It has now been found by synergy and administration studies, that antimicrobial peptides, more in particular the lactoferrin derived peptide hLF(1-11) (**Wakabayashi, H. et al**., Microbiol. Immunol. **40**:821-825 (1996)) and the histatine derived peptides DHVAR3, DHVAR4 and DHVAR5 (see e.g. EP 1 174 027, WO oo/32629, and Helmerhorst, E.J. et al., Biochem I (326) pp 39-45 (1997) all being incorporated herein by reference) are shown microbes, that are resistant to their common antibiotic drugs, sensitive therefore. However, the antimicrobial peptides are not limited to the above peptides; all microbial peptides, known in the art, are meant to be encompassed by the invention. An overview of antimicrobial peptides is given in Hancock, R.e.W. and Lehrer, R., TIBTECH (16) pp 82-88 (1998), herein incorporated by reference.

Therefore, the invention in the first place relates to the use of a combination of at least two antimicrobial agents for the preparation of a medicament for the treatment of an infection of microbes in a subject in need thereof, the microbes being resistant to the first antimicrobial agent, the second antimicrobial agent comprising an antimicrobial peptide.

Herein, the term "microbes" encompass bacteria, yeast, fungi and viruses.

The term "antimicrobial peptides", also abbreviated as AMP is known in the art. As such, AMP's form a promising potential generation of broad-spectrum therapeutics for the treatment of bacterial, fungial and viral deceases (see Hancock, Supra).

During the past two decades, over 600 antimicrobial peptides have been identified, which are an essential part of the innate defensive system of essentially all living organisms. In humans, antimicrobial and larger proteins which can release the smaller antimicrobial peptides, have been found in a variety of tissues and body fluids. Examples of AMP's are lactoferrin derived peptides and histatine-derived peptides. Further examples are disclosed in e.g. Hancock, Supra.

Human lactoferrin (hLF) is an example of an antimicrobial protein that contains antimicrobial peptides and a synergistic fungistatic effect exerted by combinations of fluconazole and human lactoferrin (hLF) was recently described (**Kuipers, M.E., et al.,** Antimicrob. Agents Chemother. **43:**2635-2641 (1999)). It has also been reported that the hyphal growth of azole-resistant *C. albicans* strains can be inhibited by combinations of bovine lactoferrin and fluconazole (**Wakabayashi, H. et al.,** Microbiol. Immunol. **40:**821-825 (1996)). Moreover, lactoferricin H, a cationic peptide released by pepsinolysis of hLF (**Bellamy, W. et al.,** Biochim. Biophys. Acta **1121:**130-136 (1992)), comprises two cationic domains (residues 2-5 and 28-31) which exhibit a candidacidal activity higher than that of the native protein (**Lamb, D.C. et al**., Antimicrob. Agents Chemother. **44:**63-67 (2000)). Furthermore, WO 01/34641 describes an effective candidacidal activity of the synthetic peptide corresponding to the N-terminus of hLF, hLF(1-11).

EP 0 629 347 and PCT/JP92/01563 describe a novel antimicrobial agent, comprising peptides, derived from a lactoferrin hydrolysate and an antibiotic. However, such an antimicrobial agent was not used for treatment of an infection of microbes that were resistant to the antibiotic, present in the said antimicrobial agent.

JP 05-610139 describes antimicrobial compositions that are effective against methicillin resistant *Staphylococcus aureus* organisms (MRSAs), comprising a combination of peptides, isolated from horseshoe crabs and of β-lactam and chloramphenicol antibiotics. The said compositions are described to have synergistic bacterial effects against MRSAs at low concentrations. However, the MRSAs were not converted to methicillin sensitivity.

The first antimicrobial agent is preferably chosen from the group, consisting of antibacterial agents, antifungial agents, antiviral agent, antiyeast agents or a combination of two or more thereof. An "antibacterial agent" is defined as a compound or a mixture of compounds, optionally combined with a pharmaceutically acceptable carrier, diluent or adjuvans, that is effective in inhibition of bacterial growth (i.e. having a bacteriostatic effect, therewith bringing bacterial growth to a hold) or in killing bacteria (i.e. having a bacterial cydic effect). Accordingly, an antifungal agent is effective in inhibiting the growth of fungi (fungistatic) or in killing fungi (fungicide). Antiviral agents inhibit further infection of virus (virostatic) or may eliminate virus infected cells, therewith eliminating the viral infection (virocide). The same is true for antiyeast agents, having a yeastostatic or yeastocidic effect. The term, encompassing the above-mentioned agents, is herein "antimicrobial agent", having correspondingly "microbistatic" effects (i.e. capable of inhibiting the growth of the microbes) or microbicidic effects (i.e. capable of killing the microbes).

The first antimicrobial agent is preferably chosen from the group, consisting of penicillin, semi-synthetic penicillin, cephem antibiotic, carbapenem antibiotics, monobactam antibiotics, aminoglycosid antibiotics, peptide antibiotics, tetracyclin antibiotics, tetracycline antibiotics, chloramphenicol, macrolide antibiotics, rifamycin, vancomycin, fosofomycin, synthetic antimicrobial agent, methicillin, polymyxin B acylclovir, or triazole agents, in particular fluconazole, or a combination of two or more thereof.

The antimicrobial peptide of the second antimicrobial agent preferably comprises an amino acid sequence, chosen from the group, consisting of:

SEQ ID NO 1: GRRRRSVQWCA    (hLF1-11)

SEQ ID NO 2: KRLFKKLKFSLRKY (DHVAR3)

SEQ ID NO 3: KRLFKKLLFSLRKY (DHVAR4)

SEQ ID NO 4: LLLFLLKKRKKRKY (DHVAR5).

SEQ ID NO 1 corresponds to the amino terminal 11 amino acids of the human lactoferrin protein, whereas SEQ ID NO 2, 3 and 4 refer to analogues of the human histatin antimicrobial peptide (Helmenhorst, Supra). As will be shown below, peptides, comprising any of the above sequences, in particular SEQ ID NO 1 and 5, are effective in conferring sensitivity for an antimicrobial agent to a microbe, resistant to the said antimicrobial agent. The antimicrobial peptide preferably has a length of less than 30 amino acids, more preferably less than 20 amino acids and most preferably less than 15 amino acids. In an attractive embodiment of the invention, the antimicrobial peptide is chosen from the group, consisting of SEQ ID NO 1, SEQ ID NO 2, SEQ ID NO 3 or SEQ ID NO 4, i.e. without any additional amino acids. However, it is to be understood that any peptide, comprising the above described sequences may comprise additional amino acids, as long as such a peptide is capable of conferring antibiotic sensitivity to the envisaged microbes. Preferably, the additional amino acids do not result in loss of sensitivity conferring function as compared with the peptides of SEQ ID NO 1, SEQ ID NO 2, SEQ ID NO 3 and SEQ ID NO 4.

Preferably, the antimicrobial peptide comprises SEQ ID NO 1 and most preferably is void of any additional amino acid sequences as discussed above. As will be shown below, the lactoferrin derived peptide hLF(1-11), which is already highly active against flucanozole resistant *Candida albicans* by itself, was found to act synergistically with fluconazole at non-Candidacidal concentrations. This finding is however not limited to hLF(1-11); therefore, in an attractive embodiment of the present invention, the antimicrobial peptide is preferably used in submicrobicidic amounts in the preparation of the medicament according to the present invention.

It has been shown that sequential administration of the first and second antimicrobial agent to a subject in need thereof, wherein the second antimicrobial agent is administered prior to the first antimicrobial agent, results in optimal resensibilisation of resistant microbes to the first antimicrobial agent. Therefore, according to the invention, the medicament comprises the first and the second antimicrobial agents as seperately administrable components that can be administered in sequential order. The skilled person will be aware of proper preparations and administration routes. The medicament can e.g. be provided in the form of tablets, (injectable) liquids, etc, or a combination thereof. The antimicrobial agents can be formulated with a suitable carrier, diluent or adjuvant as will be appreciated in the art.

The combination of the antimicrobial agents according to the invention can in particular be used against a Candida infection, in particular against a *Candida albicanes* infection. This is in particular the case when the first antimicrobial agent is an antifungal agent, preferably a triazole agent, most preferably fluconazole, whereas the antimicrobial peptide preferably comprises SEQ ID NO 1 and most preferably is hLF(1-11). Also, a combination of methicillin and an antimicrobial peptide as identified above can be used against MRSA infection.

In a second aspect, the invention relates to a medicament for treating an infection of microbes wherein the infection is a *Candida* infection, in particular a *Candida albicans* infection. As explained above, such a medicament can be used against microbial infections of microbes that are resistant to the first antimicrobial agent, the medicament rendering the said microbes sensible for the said first antimicrobial agent and therewith being capable of repelling the antimicrobial infections. The antimicrobial peptide is preferably chosen from the group, consisting of SEQ ID NO 1, 2, 3 and 4. As explained above, the antimicrobial peptide however may comprise additional amino acids. The antimicrobial peptide preferably has a length of less than 30 amino acids, more preferably less than 20 amino acids and most preferably less than 15 amino acids.

The synergistic effect of the combinatorial use of an antimicrobial peptide and an antimicrobial agent according to the present invention has even a more pronounced effect in that an antimicrobial agent, having microbistatic effect (i.e. having the capacity to inhibit the growth of microbes, but no significant effect in killing the same) may be converted to a microbicidic agent (i.e. capable of killing the microbes) when it is used in combination with an antimicrobial peptide. As will be explained in more detail below, when microbes are exposed to an antimicrobial peptide that as such is not capable of exerting a microbicidic effect (e.g. as the said peptide is given in a submicrobicidic dose or when the said peptide does not have a microbicidic effect), the microbes are killed when the microbistatic agent is added. This has even been observed when the microbes were contacted with the antimicrobial peptide before being contacted with the microbistatic agent. In numerous cases, such as when hLF(1-11), DHAVR3, DHAVR 4 or DHVAR 5 are used, the same microbicidic effect was even observed when the antimicrobial peptide was removed from the microbes before contacting the microbes with the microbistatic agent. Without being bound to any explanation, it is believed that the synergistic activity of the combination of the antimicrobial peptide with the microbistatic agent is initiated by the antimicrobial peptide, whereas the microbistatic agent is required for the effector phase, and during that phase being capable of excerting a microbicidic effect, as a result of the initiating effect of the antimicrobial peptide.

Also for the above-mentioned use, the antimicrobial peptide preferably comprises an amino acid sequence, chosen from the group, consisting of SEQ ID NO 1, SEQ ID NO 2, SEQ ID NO 3 and SEQ ID NO 4, as explained above. Further, in line with the above, the antimicrobial peptide preferably comprises less than 30 amino acids, more preferably less than 20 amino acids, and even more preferably less than 15 amino acids, the antimicrobial peptide most preferably being chosen from SEQ ID NOs 1, 2, 3 and 4.

For the conversion of a microbistatic agent to a microbicidic agent, in particular from a fungistatic to a fungicidic agent, the antimicrobial peptide is preferably used in a sub-microbicidic (or in case the microbistatic agent is a fungistatic, sub-fungicidic) amounts. As discussed above, sub-microcidic amounts are sufficient to obtain a microbicidic effect. Thus, in case the antimicrobial peptide may have microbicidic effects on its own, it may be used in small (e.g. submicrobicidic) amounts resulting in similar microbicidic function, and in cost-effectiveness.

In particular, when the fungistatic agent comprises fluconazole, the combination of fluconazole and the antimicrobial peptide can effectively be used against a Candida-infection, in particular a *Candida albicanes* infection. The most effective combinations have been observed by the combination of fluconazole and hLF(1-11).

In a third aspect, the invention relates to a method for treatment of an infection of microbes, comprising the step of administering to a person in need thereof, a therapeutically effective combinatory amount of a first and a second antimicrobial agent, the microbes being resistant against the said first antimicrobial agent, and the second antimicrobial agent comprising an antimicrobial peptide. The antimicrobial peptide is preferably administered in an amount, insufficient to excert its antimicrobial action, i.e. in submicrobicidic amounts. The first and second antimicrobial agents are administered simultaneously or subsequently. In the latter case, the second antimicrobial agent is administered before the first antimicrobial agent. Subsequent administration is preferred.

The invention will now be further illustrated by the following examples and figures there are not intended to limit the scope of the invention.

### BRIEF DESCIPTION OF THE DRAWINGS

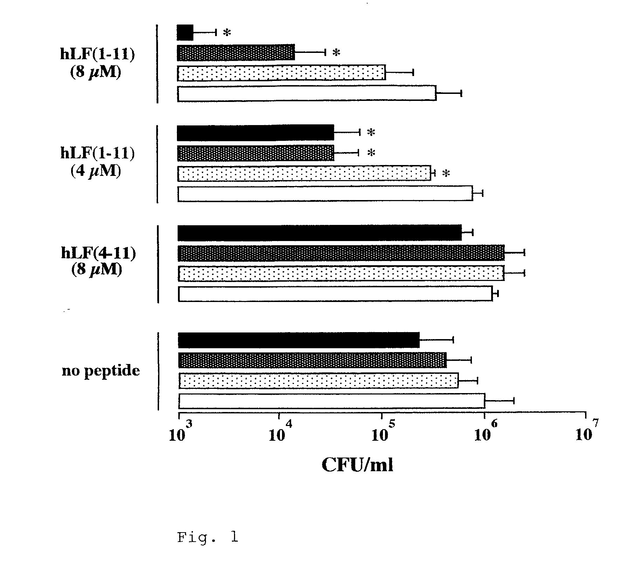

**Figure 1** - Candidacidal activity of combinations of noncandidacidal concentrations of hLF(1-11), an N-terminal peptide of human lactoferrin and 200 µg/ml fluconazole (closed bars), 150 µg/ml fluconazole (black dotted bars), 100 µg/ml fluconazole (white dotted bars) or without this triazole (open bars). The peptide hLF(4-11) served as negative control. In short, 1×10⁶ CFU fluconazole-resistant *C. albicans*/ml were incubated with combinations of hLF(1-11) and fluconazole for 2 h at 37°C and then the number of surviving *Candida* was determined microbiologically. Results are means plus SD of at least three independent experiments.
   * indicates values significantly (*P* < 0.05) different from those obtained for *Candida* not exposed to fluconazole.

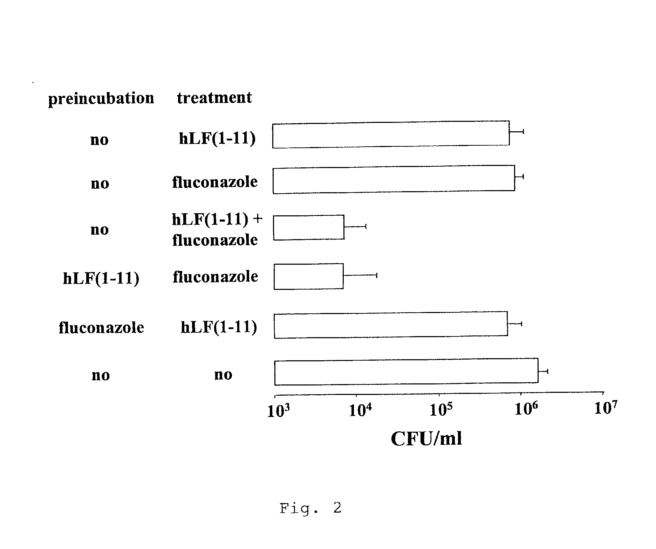

**Figure 2** - Contribution of noncandidacidal concentrations of hLF(1-11) and fluconazole to the candidacidal activity of combinations of these agents. In short, 1×10⁶ CFU *Candida*/ml were preincubated for 5 min at 37°C with (8 µM) hLF(1-11) or (200 µg/ml) fluconazole, washed and then incubated for 2 h at 37°C with (200 µg/ml) fluconazole or (8 µM) hLF(1-11), respectively. As controls, *Candida* cells were incubated for 2 h at 37°C with (8 µM) hLF(1-11), (200 µg/ml) fluconazole, the combination of (8 µM) hLF(1-11) and (200 µg/ml) fluconazole or no agent. Results are means plus SD of at least three independent experiments.

### EXAMPLES

*Lactoferrin-derived peptides.* Synthetic peptides corresponding to residues 1-11 of hLF (GRRRRSVQWCA; Mr 1,494 Da), further referred to as hLF(1-11) and, as negative control, hLF(4-11), an octamer lacking the first three N-terminal residues, and, as positive control, protegrin-1 (RGGRLCYCRRRFCVCVGR; Mr 2,161 D) were prepared and purified as described (**Nibbering, P.H. et al**., Infect. Immun. **69:**1469-1476 (2001)). Purity of these peptides usually exceeded 88%, as determined by reverse-phase high performance liquid chromatography (RP-HPLC). Stocks of these synthetic peptides at a concentration of 1 mg/ml of 0.01% acetic acid (HAc; pH 3.7), stored at -20°C were dried in a Speed-Vac (Savant Instruments Inc, Farmingdale, NY) immediately before use.

*Fluconazole*. Fluconazole (Pfizer Inc. New York, NY) was dissolved at a concentration of 1 mg/ml of dimethylsulfoxide (Fluka Chemie GmbH, Sigma-Aldrich Chemie BV, Zwijndrecht, The Netherlands) and stored at -20°C until use. From this stock solution appropriate concentrations were prepared in 10 mM of sodium phosphate buffer (pH 7.4), further referred to as NaPB. *Source of C. albicans strain.* Fluconazole-resistant *C. albicans* strain Y01-19 was purchased from Pfizer (Groton, Conn.). Yeast was identified using Candiselect (Sanofi, Pasteur, Paris, France) and confirmed by demonstration of a typical *C. albicans* pattern of sugar utilization (API, ID 32C, bioMerieux, Marcy l'Etoile, France). Fluconazole resistance (M.I.C.>256 µg/ml) was evaluated using the E-test (Oxoid Unipath Ltd., Basingstoke, UK). Yeasts were cultured overnight in Sabouraud broth (Oxoid) at 37°C and sub-cultured for 2,5 h on a rotary wheel at 37°C.

*Assay for candidacidal activity of hLF-derived peptides and fluconazole*. An in vitro assay was used to assess the candidacidal activity of hLF-derived peptides and/or fluconazole. Briefly, yeast cells were harvested in mid log-phase by centrifugation at 1,500 × g for 10 min, washed twice in NaPB, and diluted to a concentration of 1×10⁶ CFU/ml of NaPB supplemented with 2% Sabouraud broth. Equal volumes of this suspension were mixed with hLF-peptide and/or fluconazole. After a 2 h incubation at 37°C with hLF peptides and/or fluconazole the number of viable blastoconidia was determined by plating serial dilutions of each sample on Sabouraud agar. Results are expressed as colony forming units (CFU) per ml.

*Exposure of Candida to peptides and*/*or fluconazole*. To obtain some insight into the contribution of the different agents to the synergistic effect of one of the peptides hLF(1-11), DHVAR4 or DHVAR5 in combination with fluconazole, *C. albicans* cells were preincubated for 5 min with either a noncandidacidal concentration of hLF(1-11) or fluconazole at 37°C, transferred to ice for 5 min, washed twice in NaPB at 4°C, and then reincubated for 2 h at 37°C with the other agent, and finally the number of viable *Candida* was determined microbiologically.

### Candidacidal activity of combinations of noncandidacidal concentrations of hLF(1-11) and fluconazole.

Since hLF(1-11) is highly active against fluconazole-resistant *C. albicans* (Lupetti et al., Supra), noncandidacidal concentrations of this peptide were used to find out whether it, combined with fluconazole, acts synergistically in killing *Candida.* The results revealed that combinations of noncandidacidal concentrations of hLF(1-11) and fluconazole were highly active (*P* < 0.05) against the fluconazole-resistant *C. albicans*, whereas these agents alone were without effect (Fig. 1). Furthermore, the peptide hLF(4-11), which showed no candidacidal effect, when combined with fluconazole showed no significant killing activity (Fig. 1).

### Effect of preincubation with noncandidacidal concentrations of hLF(1-11) or fluconazole on the candidacidal activity of fluconazole or hLF(1-11).

To find out which of the two compounds was required to initiate this synergistic effect, *Candida* cells were preincubated for 5 min with a noncandidacidal concentration of hLF(1-11) and, after washing, incubated with fluconazole and vice versa. The results showed that the candidacidal activity of the first approach was comparable to that after co-incubation of hLF(1-11) and fluconazole for 2 h (Fig. 2). In contrast, when *Candida* cells were preincubated with fluconazole for 5 min and then exposed to a noncandidacidal concentration of hLF(1-11) for 2 h no candidacidal effect was observed (Fig. 2). Similar results were obtained when *Candida* cells were exposed to fluconazole for up to 1 h prior to the addition of the peptide (data not shown). These results suggest that the candidacidal activity of combinations of hLF(1-11) and fluconazole involves two phases: an initiator phase exerted by hLF(1-11) and an effector phase mediated by fluconazole.

From the above, it was found that the lactoferrin derived peptide hLF(1-11) which is already highly active against fluconazole-resistant *Candida albicans* by itself, acts synergistically with fluconazole at noncandidacidal concentrations. More remarkably, when this yeast was exposed to hLF(1-11) for 5 min and then incubated with fluconazole, it was killed effectively, whereas no candidacidal activity was observed when *Candida* was first incubated with fluconazole and then exposed to the peptide. These data indicate that the candidacidal activity of combinations of hLF(1-11) and fluconazole can be initiated by the antimicrobial peptide, while fluconazole is only required during the effector phase. The present invention provides that antimicrobial peptides, have unreveiled potentials by re-sensibilizing multi drug-resisitant microorganisms for their antimicrobial agent that they previously had become resistant for. In present invention, antimicrobial peptides, in particular but not limited to the lactoferrin derived peptide hLF(1-11), was able to re-sensibilize fluconazole-resistant *Candida albicans* for fluconazole by simultaneous and, in particular by sequential administration of noncandidacidal concentrations of hLF(1-11) and fluconazole.

SEQUENCE LISTING

<110> AM Pharma

<120> Use of antimicrobial peptides for potentiating the
      activity of antimicrobial agents

<130> Synergistic use of AMPs

<140>
<141>

<160> 5

<170> PatentIn Ver. 2.1

<210> 1
<211> 11
<212> PRT
<213> Artificial Sequence

<220>
<223> Description of Artificial Sequence:amino terminal
      of human lactoferrin

<400> 1
Gly Arg Arg Arg Arg Ser Val Gln Trp Cys Ala
  1               5                  10

<210> 2
<211> 14
<212> PRT
<213> Artificial Sequence

<220>
<223> Description of Artificial Sequence:synthetic
      analogue of human histatin

<400> 2
Lys Arg Leu Phe Lys Lys Leu Lys Phe Ser Leu Arg Lys Tyr
  1               5                  10

<210> 3
<211> 14

<212> PRT
<213> Artificial Sequence

<220>
<223> Description of Artificial Sequence:synthetic
      analogue of human histatin

<400> 3
Lys Arg Leu Phe Lys Lys Leu Leu Phe Ser Leu Arg Lys Tyr
  1               5                  10

<210> 4
<211> 14
<212> PRT
<213> Artificial Sequence

<220>
<223> Description of Artificial Sequence:synthetic
      analogue of human histatin

<400> 4
Leu Leu Leu Phe Leu Leu Lys Lys Arg Lys Lys Arg Lys Tyr
  1               5                  10

<210> 5
<211> 18
<212> PRT
<213> Artificial Sequence

<220>
<223> Description of Artificial Sequence:protegrin-1

<400> 5
Arg Gly Gly Arg Leu Cys Tyr Cys Arg Arg Arg Phe Cys Val Cys Val
  1               5                  10                  15

Gly Arg

The above experiments with other antimicrobial peptides, in particualr with DHAVR 3, DHVAR4 and DHVAR5, combined with numerous antibiotics as described hereinabove resulted in similar synergistic and resensibilisating effects.

## Claims

1. Use of a combination of at least two antimicrobial agents for the preparation of a medicament for the treatment of an infection of microbes in a subject in need thereof, the microbes being resistant to the first antimicrobial agent, the second antimicrobial agent comprising an antimicrobial peptide.

2. Use according to claim 1, wherein the first antimicrobial agent is chosen from the group, consisting of antibacterial agents, antifungal agents, antiviral agents, antiyeast agents or a combination of two or more thereof.

3. Use according to claim 2, wherein the first antimicrobial agent is chosen from the group, consisting of penicillin, semi-synthetic penicillin, cephem antibiotic, carbapenem antibiotics, monobactam antibiotics, aminoglycosid antibiotics, peptide antibiotics, tetracyclin antibiotics, tetracycline antibiotics, chloramphenicol, macrolide antibiotics, rifamycin, vancomycin, fosofomycin, synthetic antimicrobial agent, methicillin, acyclovir, polymyxin B or triazole agents, in particular fluconazole, or a combination of two or more thereof.

4. Use according to any of the preceding claims, wherein the antimicrobial peptide of the second antimicrobial agent comprises an amino acid sequence, chosen from the group, consisting of:

SEQ ID NO 1: GRRRRSVQWCA (hLF1-11)

SEQ ID NO 2: KRLFKKLKFSLRKY (DHVAR3)

SEQ ID NO 3: KRLFKKLLFSLRKY (DHVAR4)

SEQ ID NO 4: LLLFLLKKRKKRKY (DHVAR5).

5. Use according to any of the preceding claims, wherein the antimicrobial peptide has a length of less than 30 amino acids, preferably less than 20 amino acids, most preferably less than 15 amino acids.

6. Use according to claim 5, wherein the antimicrobial peptide is chosen the group, consisting of:

SEQ ID NO 1: GRRRRSVQWCA (hLF1-11)

SEQ ID NO 2: KRLFKKLKFSLRKY (DHVAR3)

SEQ ID NO 3: KRLFKKLLFSLRKY (DHVAR4)

SEQ ID NO 4: LLLFLLKKRKKRKY (DHVAR5).

7. Use according to any of the preceding claims, wherein the antimicrobial peptide of the second antimicrobial agent is present in the medicament in submicrobicidic amounts.

8. Use according to any of the preceding claims, wherein the medicament comprises the first and the second antimicrobial agents as separately administrable components.

9. Use according to any of the claims 1-8, wherein the infection is a *Candida* infection, in particular a *Candida albicans* infection.

10. Medicament for treating an infection of microbes comprising an antimicrobial peptide, comprising the amino acid sequence, chosen from: SEQ ID NO 1: GRRRRSVQWCA    (hLF1-11) SEQ ID NO 2: KRLFKKLKFSLRKY (DHVAR3) SEQ ID NO 3: KRLFKKLLFSLRKY (DHVAR4) SEQ ID NO 4: LLLFLLKKRKKRKY (DHVAR5), and an antimicrobial agent, chosen from the group, consisting of penicillin, semi-synthetic penicillin, cephem antibiotic, carbapenem antibiotics, monobactam antibiotics, aminoglycosid antibiotics, peptide antibiotics, tetracyclin antibiotics, tetracycline antibiotics, chloramphenicol, macrolide antibiotics, rifamycin, vancomycin, fosofomycin, synthetic antimicrobial agent, methicillin, polymyxin B, acyclovir or triazole agents, in particular fluconazole, or a combination of two or more thereof, wherein the microbes are resistant against the microbial agent.

11. Medicament according to claim 10, wherein the antimicrobial peptide is chosen from the group, consisting of:

SEQ ID NO 1: GRRRRSVQWCA    (hLF1-11)

SEQ ID NO 2: KRLFKKLKFSLRKY (DHVAR3)

SEQ ID NO 3: KRLFKKLLFSLRKY (DHVAR4)

SEQ ID NO 4: LLLFLLKKRKKRKY (DHVAR5).

12. Use of a microbistatic agent and an antimicrobial peptide for the preparation of a microbicidic agent.

13. Use according to claim 12, wherein the microbistatic agent is a fungistatic agent and the microbiotic agent is a fungicidic agent, the fungistatic agent preferably being fuconazole.

14. Use according to claim 12 or 13, wherein the antimicrobial peptide comprises an amino acid sequence, chosen from the group, consisting of: SEQ ID NO 1: GRRRRSVQWCA (hLF1-11) SEQ ID NO 2: KRLFKKLKFSLRKY (DHVAR3) SEQ ID NO 3: KRLFKKLLFSLRKY (DHVAR4) SEQ ID NO 4: LLLFLLKKRKKRKY (DHVAR5), preferably comprising less than 30 amino acids more preferably less than 20 amino acids and even more preferably less than 15 amino acids, the antimicrobial peptide most preferably being chosen from SEQ ID NO 1, 2 and 3.

15. Use according to any of the claims 12 - 14, wherein the antimicrobial peptide is used in submicrobicidic amounts.
